Europäisches Patentamt

European Patent Office

Office européen des brevets

(19)

(11) Publication number: 0 299 673

A1

## (12) EUROPEAN PATENT APPLICATION

(21) Application number: 88306191.3

(22) Date of filing: 07.07.88

(51) Int. Cl.4: **A61K 39/20 , C07K 7/06 , C07K 7/08 , C07K 7/10 , G01N 33/569 , C12N 15/00**

(30) Priority: 08.07.87 GB 8716044
04.03.88 GB 8805258

(43) Date of publication of application:
18.01.89 Bulletin 89/03

(84) Designated Contracting States:
AT BE CH DE ES FR GB GR IT LI LU NL SE

(71) Applicant: **London Biotechnology Limited
Temple Court 11 Queen Victoria Street
London E2N 4TP(GB)**

(72) Inventor: **Ho-Terry, Linda Dept of Academic
Chem. Path.
Windeyer Building Cleveland Street
London, W1P 6DB(GB)**
Inventor: **Terry, George Manfield Dept of
Academic Chem. Path
Windeyer Building Cleveland Street
London, W1P 6DB(GB)**
Inventor: **Rees, Kenneth Reginald Dept of
Academic Chem. Path
Windeyer Building Cleveland Street
London, W1P 6DB(GB)**

(74) Representative: **Stuart, Ian Alexander
MEWBURN ELLIS & CO. 2/3 Cursitor Street
London EC4A 1BQ(GB)**

(54) Rubella E1 glycoprotein antigens.

(57) A small portion of the E1 peptide of rubella virus which contains three epitopic sites is identified. This portion of the peptide is useful as a basis for standard antigenic peptides for rubella diagnosis and vaccination.

The inventors have produced the major rubella envelope antigen E1 and a fragment of it by genetic expression in E.coli. The expressed products show good reactivity with E1 monoclonal and rubella specific human Ig antibodies and also have potential as immunising agents.

EP 0 299 673 A1

## RUBELLA E1 GLYCOPROTEIN ANTIGENS

This invention relates to vaccines, bioassay procedures and materials, and more particularly to Rubella diagnosis and vaccination.

Rubella virus infection during pregnancy is associated with a risk of congenital rubella syndrome (CRS) in the fetus, the incidence of congenital malformations being highest when primary infection occurs in the first 12 weeks of gestation. At present, CRS is prevented by offering therapeutic abortion to pregnant women found to be positive for rubella specific IgM antibodies and by national vaccination programmes.

Until congenital rubella can be eradicated by an effective vaccination policy more direct control measures relying on diagnosis of rubella infection in pregnant women followed by termination of pregnancy will continue to be necessary. The diagnosis is routinely made or confirmed by the detection of maternal virus specific IgM as an indicator of recent or current primary infection. However, this is not entirely reliable, since some vaccinated people can produce IgM during reinfection even though the risk to the fetus is very low. Advances have recently been made in replacing less sensitive tests by highly sensitive immunoassays but these are not without equivocal results and in these cases, a combination of different assays may have to be used to provide a diagnosis. This can be very expensive and time consuming, and discordant (as opposed to equivocal) results can make the interpretation difficult.

Propagation of viruses in tissue culture has been invaluable in providing viral antigens required for diagnostic or immunization purposes. However, rubella virus grows to relatively low titre (Ho-Terry & Cohen 1980) and stocks are liable to accumulate defective interfering particles (Terry et al 1985). The virus particle is highly labile (Parkman et al. 1964; Chagnon & Laflamme 1964) and this adds to the difficulties in the purification of the antigen which is essential for a reliable immunoassay (Ho-Terry & Cohen 1980).

Furthermore, the Rubella antigens produced by conventional techniques are potentially infective. Rubella vaccines in current use contain live, attenuated virus. They are potentially teratogenic and are readily inactivated without proper storage. These disadvantages pose special problems in countries where contraceptive measures and cold storage facilities are limited. The vaccine cannot be given during pregnancy when protection against rubella may be most needed.

Production of rubella virus antigens is thus inefficient, costly and labour intensive, and immunoassay kits are often expensive to prepare. There is, therefore, a clear requirement for preparation of rubella antigens by alternative routes, such as chemical synthesis or genetic manipulation.

In an attempt to overcome the difficulties referred to above the present inventors, have examined an alternative approach to currently available immunoassays and vaccines, involving isolation and identification of rubella peptides which react with virus specific Ig, with a view to providing a synthetic antigen which could be used in immunoassays and which would reduce the number of equivocal results by reducing the high background shown by conventional whole virus antigens. This type of antigen would also fulfil the urgent requirement for a qualitatively and quantitatively standardised rubella antigen to determine the immune state or, by means of an IgM-specific assay, indicate current or recent infection.

Rubella virus is an enveloped, positive-stranded RNA virus and is classified in the family Togaviridae. The virus contains a single non-glycosylated capsid protein C and two glycosylated envelope proteins E1 and E2. Unlike the related alphaviruses, the E1 and E2 proteins can only be separated by employing strong denaturing conditions, which virtually destroy the immunogenic properties. The present inventors have previously reported work using monoclonal antibodies to rubella, which identified three epitopes on the E1 polypeptide, which they designated the EP1, EP2 and EP3 epitopes of E1. Antibodies to EP1 and EP2 show both haemagglutination inhibition (HI) and neutralising activity, while antibodies to EP3 show only neutralising activity (Ho-Terry et al, 1986).

Frey et al (1986) have reported the cloning of cDNA derived from the Therien strain of rubella virus, and proposed a nucleotide and deduced amino acid sequence for the E1 polypeptide. However, their disclosure provided no information as to the location and sequence of any of the major E1 epitopes. From their present work on the "Judith" strain the present inventors have obtained the E1 amino acid sequence of a U.K. virus strain from their independently derived cDNA clones and have been able to identify a portion of the E1 peptide which is likely to contain the EP1, EP2 and EP3 epitopic sites, and hence provide a basis for a standard antigenic peptide for diagnostic and other use.

The inventors have also produced the major rubella envelope antigen E1 and a fragment of it by genetic expression in E.coli. This method of antigen production is cheap and simple and the expressed products show good reactivity with both E1 monoclonal and rubella specific human Ig antibodies, and also have potential as immunizing antigens. By expressing a fragment of the E1 glycoprotein the inventors have obtained confirmation of which portion of the E1 molecule is of most immunological importance.

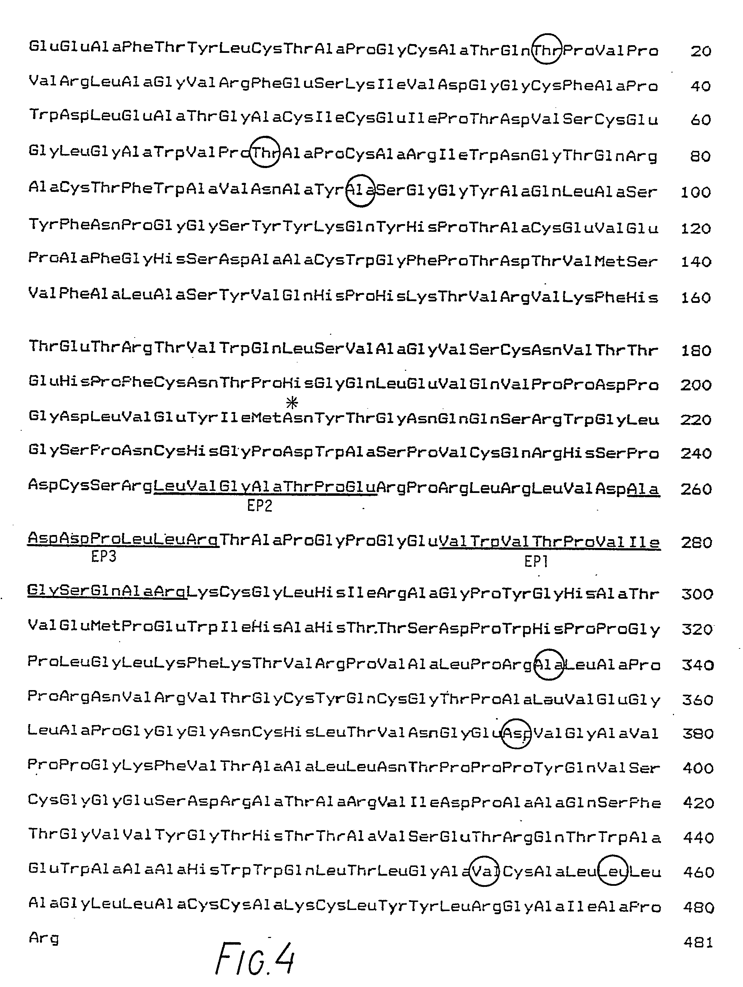

Thus, according to the present invention there is provided a peptide from the sequence 207 to 353 of Fig. 4 discussed below which binds rubella immunoglobulin (Ig) specifically, or a variant of the peptide which retains rubella Ig specific binding characteristics.

Preferably, the peptide is from the sequence 245 to 285 of Fig. 4 which is the smallest portion of E1 which the inventors believe to contain all three of the epitopic sites EP1, EP2 and EP3. The invention also provides variants of the preferred peptides which retain rubella Ig specific binding characteristics.

Particularly preferably, the peptide includes one or more of the sequences 245-251, 260-266 and 274-285 of Fig. 4 or is a variant of such a peptide which, again, retains rubella Ig specific binding characteristics.

In addition the invention provides peptides which comprise at least part of the various sequences referred to above and which bind rubella Ig specifically, or variants of these peptides which retain rubella Ig specific binding characteristics. Preferably, said peptides or variants are substantially free of natural type glycosylation.

Thus, the invention includes, for example, fusion proteins of a part of E1 with another peptide. The other peptide is preferably one which facilitates the purification of the fusion protein by binding to an attachment ligand. For example the various peptides mentioned above can be produced as fusion proteins with a further peptide capable of binding an Ig. Protein A is a suitable further peptide.

The various peptides of the invention can be employed in assays for rubella Ig, for example in an indirect ELISA as described below. In that case, an unlabelled peptide of the invention is used to bind rubella antibody which is subsequently detected by a signal producing system.

Another possibility is that a peptide of the invention be labelled, the label being part of a signal producing system. For example, the label could be an enzyme which takes part in reactions of the signal producing system. Alternatively, the peptide could carry an enzymically inactive small fragment of a primary enzyme which can be conjugated with a complementary enzymically inactive protein fragment so as to produce an active primary enzyme. Such a system is described in GB-2156581-A where ribonuclease S-peptide is described for use as the small fragment of the primary enzyme, and is conjugated with the complementary S-protein fragment. The active primary enzyme catalyses a reaction whose product leads to, or is, an essential coenzyme or prosthetic group for a second enzyme which catalyses a secondary reaction leading to a detectable result and indicating the presence of an analyte. In the case of the present invention the analyte detected would be rubella Ig.

The invention also provides peptides carrying an enzyme or an enzymically inactive small fragment for use in the method mentioned above.

Peptides of the present invention may react both with IgG and IgM for rubella. Peptide- bound IgG or IgM can then be detected specifically in an indirect test using anti-IgG or anti-IgM. However, if rubella IgM alone is determined it may be desirable to separate total IgM in a test serum from other material such as IgG before assaying for rubella IgM. The invention therefore also provides an assay for rubella IgM comprising separating total IgM from a sample to be assayed, binding a peptide of the invention to rubella IgM in the separated total IgM and detecting the bound peptide by antibody specific to that peptide. Total IgM may be separated from a clinical sample using an anti-human IgM antibody on a support. for example as described in EP-0162533-A. The IgM captured on the support is then reacted with a peptide of the present invention before a labelled antibody to the peptide, for example a monoclonal antibody is used to detect the bound peptide.

It may be desirable to increase the valency of the peptides of the invention for use in this method to assure that sites are available on the peptide for binding the detecting antibody. Thus, the peptides could be aggregated artificially using glutaraldehyde, linked to B.S.A., or expressed as multiple copies to ensure polyvalency.

Work by the present inventors has indicated that peptides of the present invention possess some utility as vaccines. The peptides are suitably used in injectable form e.g. in a sterile buffered solution. suspension or emulsion with or without adjuvant. In the case of the smaller peptides it is preferable to employ an adjuvant, and adjuvants are highly desirable where the vaccine is for administration to human patients.

In the accompanying drawings:

Fig.1 shows ion-exchange chromatography of Staph. aureus V8 protease cleaved rubella virus peptides in a Mono-Q column;

indicates immunoreactivity expressed as a ratio of reaction of a rubella antibody positive and a negative serum;

- - - - indicates ³H fucose radioactivity;

....... indicates the NaCl elution profile.

EP 0 299 673 A1

Fig.2 shows molecular sieve chromatography on a Superose 12 column

(a) Fraction 11 from ion- exchange.

(b) Rubella peptides derived from trypsin digestion of the 13K fragment from (a).

Molecular weight markers used included bovine serum albumin (66K), carbonic anhydrase (29K), cytochrome c (12.4K) and aprotinin (6.5K).

Fig. 3 presents the information in Fig. 2 in the form of a peptide map.

Fig. 4 shows the complete amino acid sequence of rubella E1 glycoprotein.

* Indicates the position in the E1 amino acid sequence, of a potential glycosylation site found to contain oligosaccharide with molecular weight equivalent to fourteen amino acids. Amino acids circled represent differences from the strain used by Frey et al. (1986).

Fig. 5 is a schematic representation of the recon struction of rubella E1 nucleic acid sequences. S indicates the signal sequence, T indicates the first termination codon in the reverse orientation, and the stippling indicates overlapping sequences which have been removed. The hatching indicates a portion of the reconstructed E1 nucleic acid sequence bound by two Rsal restriction sites.

Fig. 6 shows the ELISA immunoreactivity of cellular extracts of expressed pRIT2 (▲--▲), pRIT2 R (■----■) and pRIT2/E1 (●————————————————●), using rubella E1EP1 monoclonal. antibodies. Vertical bars indicate the range of 5 replicate ELISA readings;

Fig. 7 shows polyacrylamide gel electrophoresis (PAGE) and Western Blot of cellular extracts of expressed pRIT2 (E), pRIT2/R(R), pRIT2/E1(E1) and pRIT2/eps(eps). The results are shown diagrammatically. Rubella E1EP1 monoclonal antibody was used to specifically detect rubella antigens on the Western Blot. Unblotted polypeptides (PAGE) were stained with Coomassie blue. Molecular weight markers (M) were run in an adjacent lane. The most prominent protein bands on PAGE in the rubella antigens are marked "*".

Fig. 8 shows reactivity in an ELISA test, of the smaller E1 antigen expressed by pRIT2/eps with three monoclonal antibodies which define 3 distinct epitopes on the rubella E1 protein.

## (A) LOCATION OF EPITOPIC SITES

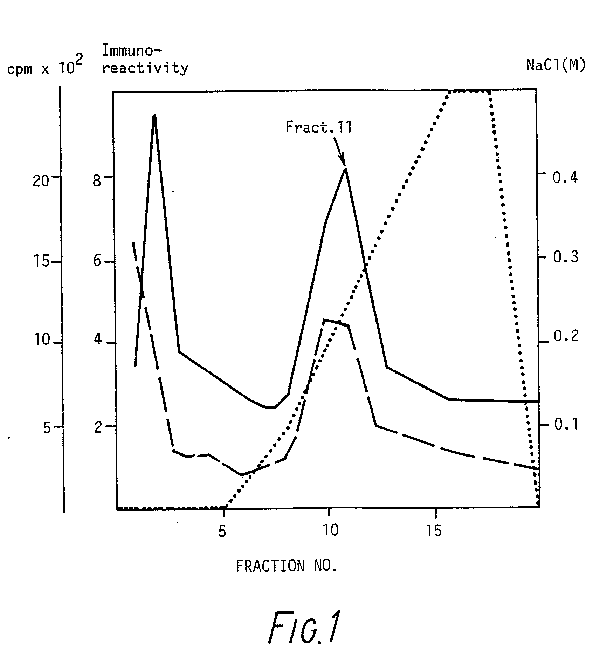

Rubella virus strain Judith was used throughout. $^3$H-fucose labelled purified rubella virus containing 6 x $10^5$ haemagglutination units (HAU) was digested with Staph. aureus V8 protease (200 μg/ml) (Sigma) in 20 mM Tris-HCl (pH 8.0) containing 0.1% reduced Triton X-100 (Aldrich), and 50 mM CaCl$_2$ for 16 hours at 37°C. Undigested viral protein aggregates were removed by centrifugation in a bench microcentrifuge for 15 minutes and the peptides separated by FPLC on a Mono-Q ion-exchange column (Pharmacia) by elution with a 0-0.5 M NaCl gradient in 20mM Tris-HCl (pH 8.6), 0.1% reduced Triton X-100 (Fig. 1). Two labelled antigenic components, designated A and B, were isolated. Component A eluted with the void volume and was found to contain various aggregates composed of intact E2 and intact or partially degraded E1. Component B was also heterogeneous and eluted at around 0.25M NaCl.

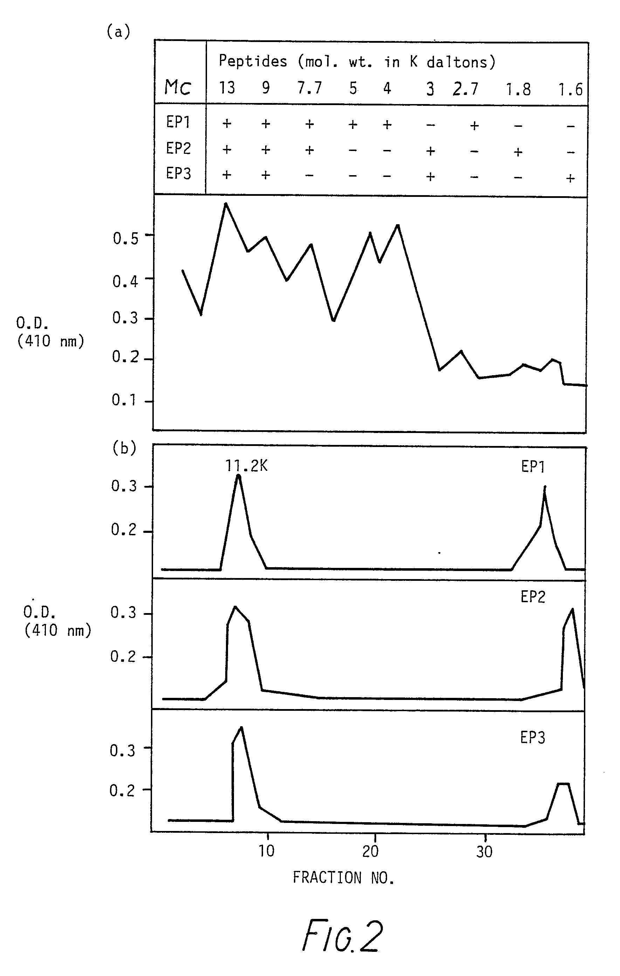

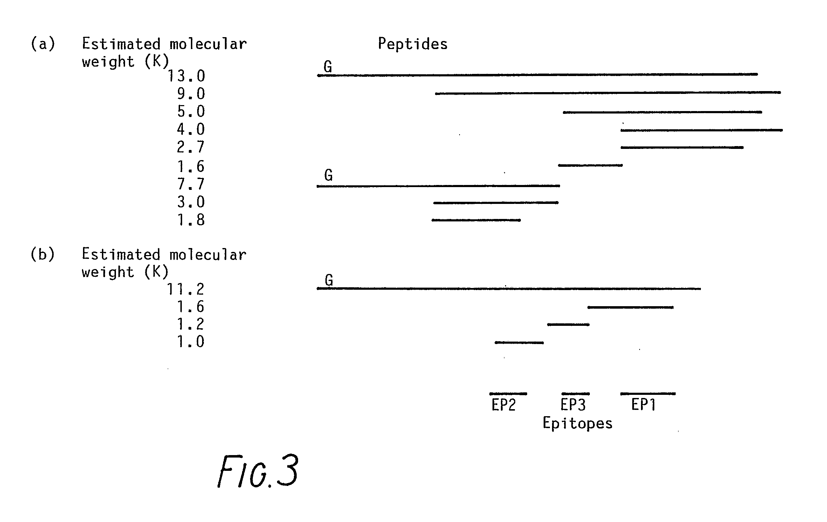

The fraction containing most antigenic activity (fraction 11) of fraction B was chosen for further analysis by FPLC molecular sieve chromatography on a Superose 12 in a HR10/30 column (Pharmacia). Elution was carried out with 100 mM Tris-HCl (pH 8.6) containing 0.1% reduced Triton X-100. Protein markers of known size were used to estimate the sizes of eluted peptides. The antigenic peptides separated by chromatography were located by reaction with a pooled human serum in an indirect ELISA (Enzyme Linked Immunosorbent Assay) according to the method of Vaheri and Salonen (1980) and their reactivity with 3 rubella specific monoclonal antibodies directed against individual E1 epitopes (Ho-Terry et al. 1986) was studied in a similar way. Reactivity of the various fractions with each of the monoclonal antibodies is summarised in Figs. 2a and 3a. The results summarised in Fig. 2a identify a $^3$H-fucose containing 13K peptide which reacts with all 3 rubella specific monoclonal antibodies. This 13K peptide was also found to react with 6 out of 7 acute sera positive for rubella specific IgM (Table 1) and 7 out of 7 sera positive for rubella specific IgG (data not shown}.

4

TABLE 1

| Sera | Rubazyme-M(O.D.$_{492}$) | Reactivity with 13K-V8 |
|---|---|---|
| High positive control | 1.562 | + |
| Low positive control | 0.510 | + |
| Negative control | 0.131 | - |
| IgM positive serum 1 | 1.520 | + |
| IgM positive serum 2 | 1.723 | + |
| IgM positive serum 3 | 1.235 | + |
| IgM positive serum 4 | 0.714 | + |
| IgM positive serum 5 | 0.670 | - |
| IgM positive serum 6 | 0.576 | + |
| IgM positive serum 7 | 0.825 | + |

Table 1. Reactivity of the 13K-V8 peptide with rubella IgM positive human sera in an indirect ELISA. The test sera were all defined as IgM positive using a Rubazyme-M kit (Abbott Labs.).

Since most of the antigenic determinants are associated with the 13K peptide, it was chosen for further analysis by cleavage with trypsin. The 13K peptide was digested with trypsin (Sigma) at 200 μg/ml in 20 mM Tris-HCl (pH 8.0) containing 0.1% reduced Triton X-100 and 100 mM CaCl₂ for 16 hours at 37° C and fractionated on the Superose column as before. The reactivity of the tryptic peptides derived from the glycosylated 13K peptide with the same rubella E1 monoclonal antibodies is shown in Fig. 2b and Fig 3b. The fractions eluted included one fucose containing peptide of molecular weight 11.2K which reacted with all three monoclonal antibodies. In addition, non-glycosylated peptides of molecular weights 1.6-1.8K were also found which reacted specifically with individual monoclonal antibodies (Fig. 2b and Fig. 3b).

In order to estimate the size of the oligosaccharide, the 13K peptide was digested to completion with pronase (200 μg/ml) and the $^3$H-fucose labelled oligosaccharide separated on the Superose column as before. It was found to have a molecular weight of 1.4-1.8K.

In order to locate the antigenic peptides on the complete E1 map the following procedure was carried out. Rubella 40s RNA was extracted from purified virus strain Judith with phenol-chloroform (Maniatis et al 1982) , precipitated by ethanol and purified by centrifugation on a 10% (w/w) to 50% (w/w) sucrose gradient in TES (100mM NaCl, 10mM Tris pH 7.5. 1mM EDTA) for 180 minutes at 40K rpm at 20°. The virion RNA collected was reverse transcribed as described by Maniatis et al. and the second strand synthesized as described by Okayama and Berg (1982). The cDNA was tailed with oligo-dC and cloned into the Pst 1 site of oligo-dG tailed pATI53. The insert was excised, separated from the plasmid DNA by electrophoresis on 1% agarose gel and subcloned into M13mp18. Overlapping subclones were sequenced by the dideoxy chain termination method of Sanger et al. (1977). The deduced amino acid sequence is shown in Fig. 4.

The known cleavage sites for Staph. aureus V8 protease and trypsin allow the various peptides isolated to be located within the derived amino acid sequence. Staph. aureus V8 protease cleaves the carboxyl terminal side of aspartic acid and glutamic acid and trypsin cleaves the carboxyl terminal side of lysine and arginine. Thus, the Staph. aureus V8 protease and trypsin cleavage sites located the glycosylated 13K peptide at amino acids 202 to 305 in the predicted amino acid sequence and the monoclonal antibodies Figs. 2 and 3), further localized the previously characterized rubella E1 epitope sites to within amino acids 245 to 285. The final map obtained is shown in Fig. 3. That the rubella E1 antigenic sites are most likely to be located in this 41 amino acid region is also supported by (1) the high hydrophilicity of this region and (2) the inventors' previous finding that haemagglutination inhibition (HAI) antibodies (i.e. those which react with EP1 and EP2) react with the protein and not the oligosaccharide component of rubella envelope glycoprotein E1 (Ho-Terry & Cohen, 1984; Ho-Terry et al, 1986). The localisation of the rubella E1 epitopes into a small antigenic peptide of 41 amino acids suggests that it is a good candidate for (1) a potentially fully characterisable standard rubella specific ELISA antigen and (2) a synthetic vaccine.

It was possible, from a-knowledge of a) the sizes of the fragments from enzyme digestion, b) the reactivity of each of the fragments with monoclonal antibodies, and c) Staph. aureus V8 protease and trypsin cleavage sites to putatively locate the epitopic sites within the E1 amino acid sequence. Thus, epitope EP1 or a part thereof is located between amino acids 274 to 285, EP2 is located between amino acids 245 to 251 and EP3 is found between amino acids 260 and 266.


PRODUCTION OF ANTIGEN

Comparison of cDNA sequence data and peptide mapping data described above suggests that the three epitopes previously characterised on E1 are located close together and near the middle region of the protein. This and earlier data (Ho-Terry & Cohen 1984) suggest that the epitopes may be continuous, rather than conformation-dependent, and do not depend on glycosylation of the protein. Rubella E1 is, for these reasons, a good candidate for expression in an E.coli system.

E1 glycoprotein has previously been expressed in cos cells after transfection with SV40 derived expression vector including rubella virus cDNA (Nakahasi et al 1986). The impure material produced was not shown to have any use in rubella diagnosis. The present inventors chose to express E1 in E.coli using a protein A fusion vector in order to facilitate purification of the antigen. Producing the antigen in a bacterial system also enables greater quantities to be produced that are possible using mammalian cells.


(1) Reconstruction of rubella E1 gene and subcloning

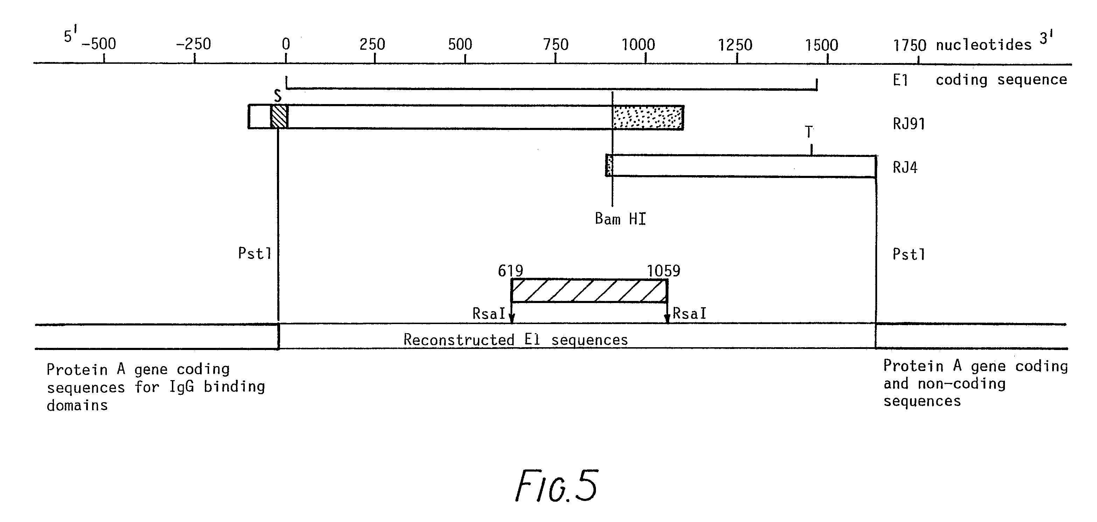

Fig. 5 shows the scheme by which 2 overlapping cDNA clones containing rubella E1 nucleic sequences were coupled. Briefly, overlapping cDNA clones designated RJ91 and RJ4, obtained by procedures described previously (see above and Terry et. al (1986)), containing the 5´ and 3´ E1 sequences respectively and a single Bam HI site in the overlapping region were digested with Pst I and Bam HI. The 5´ and 3´ Pst I-Bam HI fragments were isolated by electrophoresis on agarose gels and ligated with Pst I digested pAT153.

The 1.55kb DNA fragment containing the complete rubella E1 nucleic acid sequence was then subcloned into the Pst I site of the protein A gene fusion vector, pRIT2T Pharmacia) which contains the lambda rightward promoter. The resulting vector, pRIT2/E1, contains the coding sequence for the last 10 amino acids of the 19 amino acid E1 signal peptide, the whole of the E1 polypeptide itself (480 amino acids), and a 62 base untranslated region (plus 3´ poly A and oligo dC tails resulting from the cloning procedure), fused in frame to the 3´ end of the coding sequence for the immunoglobulin binding sites of protein A.

pRIT2/R, containing the same rubella cDNA sequences but in the reverse orientation. and pRIT2 itself (no insert) were also used as controls.

Each resulting vector was used to transform E.coli strain N4830-1 (also obtained from Pharmacia) which expresses the temperature sensitive lambda cIts 857 repressor. Transformation was carried out as described by Hanahan (1983).

Fusion proteins were expressed in E.coli by a temperature shift from 30°C to 42°C to inactivate the lambda repressor. After 90 minutes the cells were lysed by sonication (Soniprep 150, MSE) followed by removal of cell debris by centrifugation at 15000rpm for 20 min and purification of the fusion protein by affinity chromatography on an IgG Sepharose 6FF column (Pharmacia). The protein was adsorbed to the column in 50mM tris/HCl pH 7.6, 150mM NaCl. 0.05% Tween 20 (TST) and (after extensive washing in the same buffer but with the NaCl omitted (TT)) eluted in 0.5M lithium diiodosalicylate (LIS) and desalted by chromatography on a Sephadex G25 column using TT as eluent.

In order to express a fragment of E1, pRIT2/E1 was digested with Rsal and a 0.44kb restriction fragment coding for E1 amino acids 207-353, and expected to include the 3 E1 epitopes identified above was isolated by agarose gel electrophoresis and subcloned in the correct orientation and reading frame by blunt end ligation into the Pst1 site of pRIT2 after T4 DNA polymerase treatment. This plasmid was designated pRIT2/eps. The plasmid was introduced into E.coli, and the polypeptides expressed and purified as described above.


(2) Detection and Purification of the fusion proteins

Soluble proteins extracted from E.coli transformed with pRIT2, pRIT2/R, or pRIT2/E1 by sonication after induction at 42°C were used as crude antigens in an indirect ELISA test to determine whether antigenic. rubella specific, proteins could be synthesised in E.coli.

ELISA was carried out by attaching the bioengineered antigen (4°C, overnight) to wells of ELISA trays

6

precoated with purified rabbit IgG (Sigma) and vacant protein A IgG binding sites were blocked by incubation with 0.5% rabbit IgG for 3 hrs at room temperature. Subsequent incubations were all at room temperature and were for 1 hr and 1.5 hr for the first and second antibodies respectively. Either polyclonal (Sigma) or monoclonal (BioScot) alkaline phosphatase conjugated anti-human IgM was used. Normal rabbit serum was added to all antibody diluents to a final concentration of 1/100 in order to block vacant protein A IgG binding sites. Appropriate controls were included to ensure that complete blocking was achieved. P-nitrophenyl phosphate (Sigma) in 9.7% diethanolamine, pH 9.8 was used as substrate and the enzyme reactions were stopped after 30 minutes and read at 410nm.

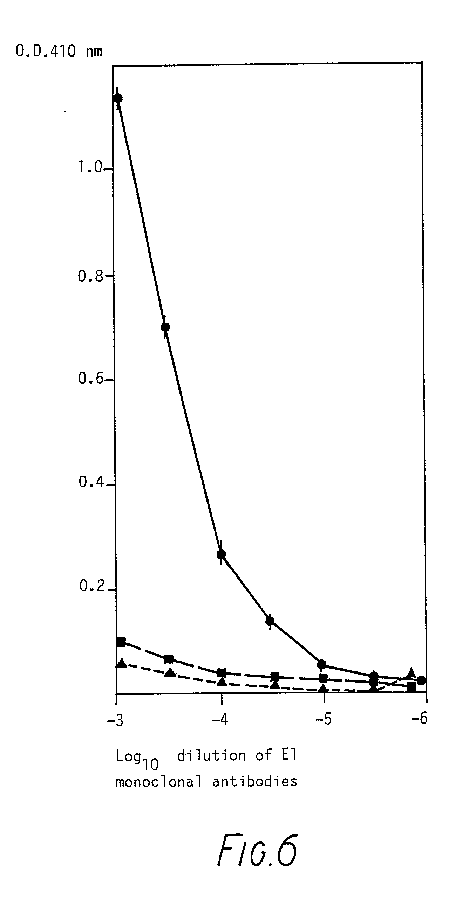

Rubella specific antigens were detected only in the extract from cells transformed by pRIT2/E1 (Fig. 6) using monoclonal antibody which defines the EP1 epitope on E1. The monoclonal antibody did not react with protein expressed by pRIT2 (the gene product in this case being simply the protein A immunoglobulin binding sites) or by pRIT2/R (the gene product in this case being the portion of protein A which contains the IgG binding domains linked to a nonsense peptide of probably 32 amino acids synthesized from the 3' end of the insert up to the first termination codon in the reverse orientation).

### (3) Western blotting

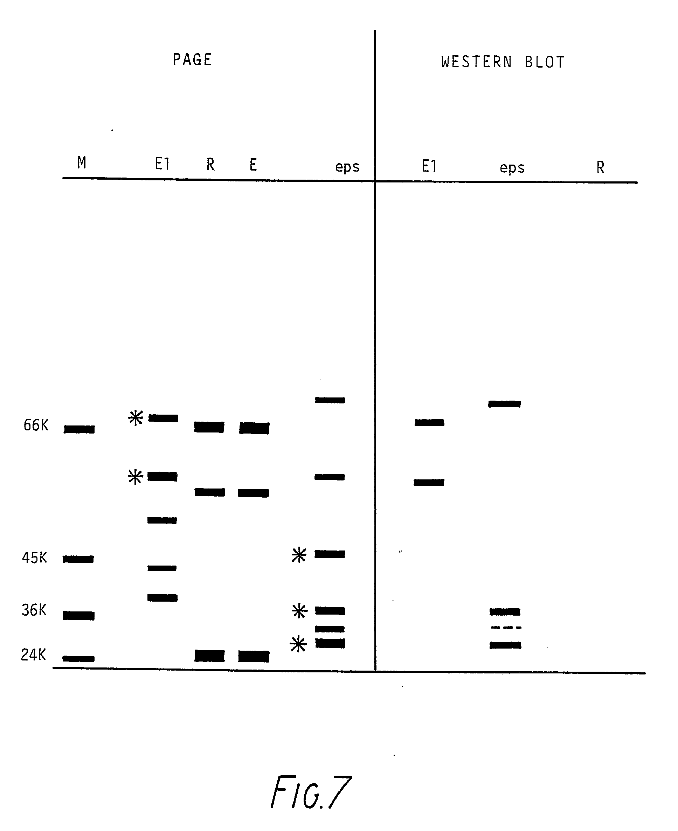

Fusion proteins expressed by pRIT2, pRIT2/R pRIT2/E1 and pRIT2/eps were purified by affinity chromatography using LIS for elution and analysed by Western Blotting. The results of this analysis are shown diagrammatically in Fig. 7.

Electrophoresis was carried out in SDS-containing 7.5% polyacrylamide slab gels and transblotting was onto nitrocellulose as previously described (Ho-Terry et al. 1986). Filters were blocked with 3% gelatin and 0.5% rabbit IgG and rubella specific proteins were detected using previously characterised monoclonal antibodies which define 3 epitopes on E1 ($E1_{EP1-3}$) (Ho-Terry et al. 1986). Reaction of the immunoreactive bands with rubella E1 monoclonal antibodies (in the presence of rabbit IgG) was followed by reaction with an anti-mouse horseradish peroxidase Immun-Blot kit (bio-Rad). Molecular weight markers (Sigma) were bovine serum albumin (66K), ovalbumin (45K), glyceraldehyde 3-phosphate dehydrogenase (35K) and trypsinogen (24K).

Only pRIT2/E1 and pRIT2/eps directed the expression of polypeptides reacting with E1 specific monoclonal antibodies. The expressed antigen appeared as polypeptide species with molecular weights 68kD and 59kD in the case of pRIT2/E1 and 40kD and 30kD in the case of pRIT2/eps. In the latter case two faint bands were also observed at 33kD and 75kD. Since the expected molecular weight of the full length E1 fusion protein is about 78kD (26.5kD for protein A and 51.5kD for unglycosylated E1), premature termination of translation or post-translational proteolysis must have occurred. This does not seem to have occurred in the case of the shorter fusion protein (or at least it is only partial) which should be about 42kD. The longest polypeptide detected would represent expression of the E1 protein up to around amino acid 388 (81% of full length). Polypeptide bands were discrete and no evidence of tailing or random cleavage was found. The presence of 2 major rubella specific polypeptides with a molecular weight difference of 10kD in each fusion protein preparation does, however, suggest that the protein A moiety is susceptible to proteolysis.

### 4) Immunoreactivity of purified pRIT2/eps expressed antigen

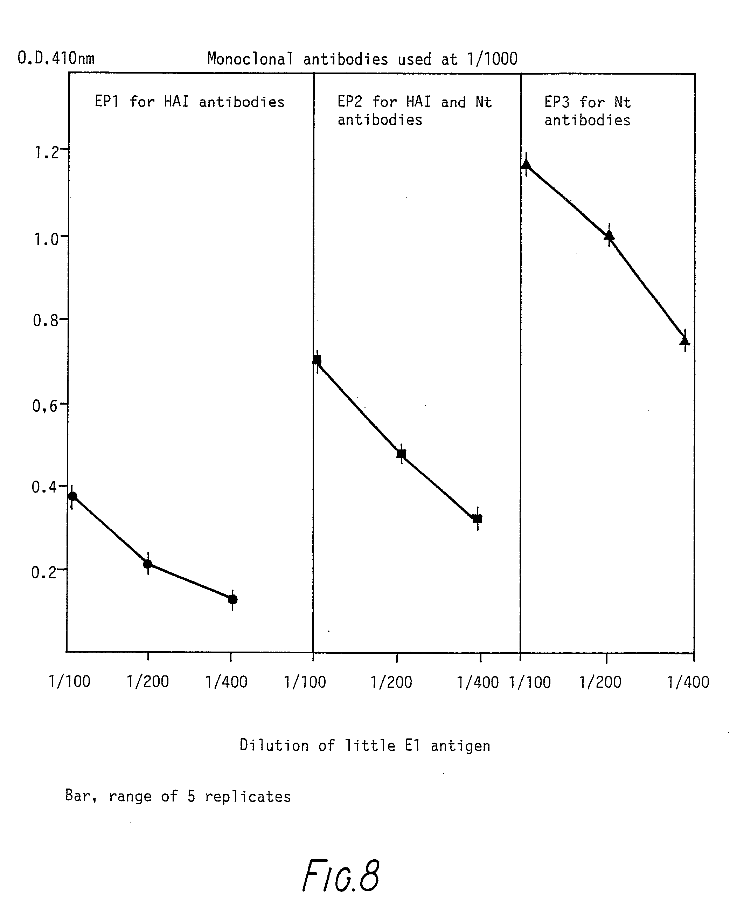

The results described above show that the fusion protein expressed by this plasmid contains at least one of the epitopes recognised by monoclonal antibodies of the present inventors. The protein was therefore tested against each monoclonal specificity to see whether all three epitopes were expressed in antigenic form. The results obtained (Fig. 8) indicate that they are, confirming the previous findings that all three epitopes are located close together on the E1 polypeptide. The reactivity of $E1_{EP1}$ was much lower than the others and this was in contrast to the results using native viral antigen where this epitope is the most reactive. This suggests that $E1_{EP1}$ is, in fact, a discontinuous epitope and its reactivity may be, at least partially, conformation dependent.

(5) Immunoreactivity of pRIT2 E1 expressed antigen with human sera

The ability of the E1 antigen expressed by pRIT2 E1 to detect rubella specific IgM antibodies in human sera is shown by the data presented below in Tables 2a and 2b. In the tables + indicates that the assay was positive for rubella specific IgM, - indicates that the assay was negative for rubella specific IgM, and + - indicates an equivocal result.

The sixteen sera were examined for rubella specific IgM using four different serological tests. Two were commercially available tests using virus derived antigens- namely the Rubenz -M test which is an IgM capture assay and the Rubazyme -M test which is an indirect ELISA. The third used the bioengineered E1 antigen in an indirect ELISA which was carried out as described above. Fourthly, the sera were tested in an indirect ELISA using purified rubella virus antigen according to the method of Ho-Terry et al (1984).

Table 2a

| Immunoassay antigen | | number of sera |
|---|---|---|
| Engineered E1 | purified virus | |
| + | + | 5 |
| - | - | 8 |
| + | + /- | 1 |
| - | + - | 1 |
| - | + | 1 |

Table 2b

| Immunoassay antigen | | | number of sera |
|---|---|---|---|
| engineered E1 | virus | | |
| | RubenzM | RubanzymeM | |
| + | + | + | 5 |
| - | - | - | 6 |
| + | + | - | 1 |
| - | + | - | 3* |
| - | + /- | - | 1** |

*, 13 weeks after illness, after asymptomatic infection, and 17 weeks after illness
**, 13 weeks after contact

The results were obtained by testing during pregnancy, sera obtained from patients with clinically diagnosed, laboratory confirmed rubella using the bioengineered E1 antigen in comparison with alternative tests. The results are presented to show that the bioengineered antigen is capable of detecting rubella specific IgM antibodies in huma sera and gives results comparable to existing test antigens. Differences can be accounted for by since: 1) some sera were collected late after infection and would therefore have very low IgM levels; and 2) further work may be required to determine the optimum test conditions where E1 antigen is used.

(6) Immune response of rabbits immunised with rubella envelope component and antigen expressed by pRIT2/E1

Three rabbits were used and each received three injections without adjuvant according to the scheme indicated below.

```
Bleed                    1st          2nd                    3rd


                          ↑            ↑                      ↑
           1      |2      3      |4     5      6      7      |8

weeks      ↓             ↓            ↓


Injection  1st          2nd          3rd
```

Rabbit A was sequentially intravenously injected with 20,000 units of rubella virus haemagglutinin, 20µg of fusion protein containing E1 and 20,000 units of haemagglutinin. Rabbit B was injected with 20µg of fusion protein containing E1 throughout and Rabbit C served as negative control.

The antibody response from the immunised rabbits is shown in Table 3. ELISA was against purified rubella virus strain Judith according to the method previously described by Ho-Terry et al. 1984. Virus neutralisation was carried out in RK13 cells using a focus-reduction assay (Taylor-Robinson & Ratcliffe 1969). The end-point was read as the serum dilution giving 50% focus reduction. The indicator virus used was RS, a wild type rubella isolate. Haemagglutination inhibition (HAI) test, using Rubella virus strain Judith as antigen was carried out as described by Liebhaber (1970).

Table 3

| Table 3a | | | | |
|---|---|---|---|---|
| Injection | Rabbit A | | | |
| | Ag | ELISA | HAI | Nt |
| 1st | 20000HAU | -4 | 1/32 | <1/10 |
| 2nd | E1(20ug) | -6 | 1/128 | 1/20 |
| 3rd | 20000HAU | -9 | >1/1024 | 1/60 |
| Table 3b | | | | |
| Injection | Rabbit B | | | |
| | Ag | ELISA | HAI | Nt |
| 1st | E1(20ug) | -2 | <1/4 | <1/10 |
| 2nd | E1(20ug) | -3.5 | <1/4 | <1/10 |
| 3rd | E1(20ug) | -5 | <1/4 | 1/25 |
| Table 3c | | | | |
| Injection | Rabbit C | | | |
| | Ag | ELISA | HAI | Nt |
| 1st | None | <-1 | <1/4 | <1/10 |
| 2nd | None | <-1 | <1/4 | <1/10 |
| 3rd | None | <-1 | <1/4 | <1/10 |

The ELISA results show that E1 expressed in E.coli was immunogenic in rabbits and was capable of stimulating a low level of neutralizing antibodies after repeated immunisations. However, no HAI antibodies were produced even after three injections. In contrast, this antigen appears to have stimulated a 4-fold rise in HAI antibody titre in the rabbit which had been previously primed with native rubella antigens. In addition, neutralising antibodies. which were undetectable after a single immunisation with virion antigens, were raised to detectable levels after boosting with E1 antigens.

Failure of the bio-engineered E1 antigen to elicit HAI antibody production in rabbits supports the view, mentioned above that $E1_{EP1}$ is partially conformation dependent since this epitope is defined by a monoclonal antibody which has high HAI activity.

That the bio-engineered antigen, by itself, stimulated mainly non-neutralising antibodies. but was able to stimulate both neutralising and HAI antoibodies against wild type virus in a rabbit which had been previously sensitized with wild type rubella virus suggests that the bio-engineered antigen could be used as a non-infectious subunit rubella vaccine which could be used as a supplement to the routine vaccination programme in providing protection for sero-negative woman at times immediately before and during pregnancy. At present this is not possible since the vaccine in current use contains live virus and cannot. therefore, be used at these times.

References

Ho-Terry & Cohen (1980). Arch. Virol. 65, 1-13
Terry, Ho-Terry, Londesborough & Cohen (1985). Arch Virol 86, 29-36
Parkman, Buescher, Artenstein, Mcown, Mundon & Druzd (1964) J. Immunol. 93, 595-607
Chagnon & Laflamme (1964) Can. J. Microbiol. 10, 501-503
Ho-Terry, Terry, Cohen & Londesborough (1986) Arch. Virol. 90, 145-152
Frey, Marr, Hemphill & Dominguez (1986) Virology 154, 228-232
Vaheri & Salonen (1980) J. Med. Virol. 5, 171-181
Sanger et al (1977) PNAS USA 74, 5463-5467
Ho-Terry & Cohen (1984) Arch. Virol 79, 139-146
Nakahasi, Meyer and Liu (1986) J. Biol. Chemm. 261, 16616-20
Terry, Ho-Terry, Warren, Rodeck, Cohen & Rees (1986) B.M.J., 292, 930-33
Maniatis, Fritsch & Sambrook (1982) Molecular Cloning. A laboratory manual. Cold Spring Harbour, New York.
Okayama & Berg (1982) Mol. Cell. Biol., 2, 161-170 Hanahan (1983) J. Mol. Biol., 166, 557-580
Ho-Terry, Cohen & Terry (1984) J. Med. Microbiol., 17, 177-180
Taylor-Robinson & Ratcliffe (1969) International Symposium on Rubella Vaccines. London 1968, Symp. Series Immunobiological Standard., 11: 117-120
Liebhaber (1970) J. Immunol., 104, 826-834

## Claims

1. A peptide from the sequence 207 to 353 of Fig. 4 which binds rubella immunoglobulin (Ig) specifically, or a variant of said peptide which retains rubella Ig specific binding characteristics.

2. A peptide comprising at least part of the sequence 207 to 353 of Fig. 4 which binds rubella Ig specifically, or a variant of said peptide which retains rubella Ig specific binding characteristics wherein said peptide is substantially free of natural type glycosylation.

3. A peptide according to claim 1 or claim 2 from the sequence 245 to 285 of Fig. 4, or a variant of said peptide which retains rubella Ig specific binding characteristics.

4. A peptide according to any one of the preceding claims including at least part of one or more of the sequences 245-251, 260-266 and 274 to 285 of Fig. 4, or a variant of said peptide which retains rubella Ig specific binding characteristics.

5. A peptide according to any one of the preceding claims for use as a vaccine against rubella.

6. A vaccine comprising a peptide of any one of claims 1 to 4 in injectable form.

7. A method of assaying for rubella Ig the method involving use of a peptide of any one of claims 1 to 4 as a probe.

8. A method according to claim 7 wherein the peptide is used as an antigen in an ELISA assay specific for IgG or IgM.

9. A method of detecting rubella Ig in a sample medium involving the use of a peptide according to any one of claims 1 to 4 wherein the peptide carries an enzyme label, said enzyme label catalysing a reaction which leads to a detectable result.

10. A method of detecting rubella Ig in a sample medium involving the use of a peptide according to any one of claims 1 to 4, said peptide carrying an enzymically inactive small fragment of a primary enzyme; the addition of a complementary enzymically inactive protein fraction of the enzyme so as to conjugate to said small fraction and produce an active primary enzyme; and the performance of a reaction catalysed by said enzyme. leading to a detectable result; and wherein said primary enzyme catalyses a reaction which converts a substrate into a primary product which itself, or as a subsequent product produced by a further reaction or series of reactions initiated by said primary product is an essential component of a second enzyme which is thereby completed and catalyses a reaction leading to a detectable result.

11. A peptide of any one of claims 1 to 4 carrying an enzyme or enzymically inactive small fragment for use in the method of claim 9 or claim 10.

12. An assay for rubella IgM comprising separating total IgM from a sample to be assayed, binding a peptide of any one of claims 1 to 4 to rubella IgM in the separated total IgM and detecting the bound peptide by antibody specific to that peptide.

13. A fusion protein comprising a peptide of any one of claims 1 to 4 and a further peptide.

14. A fusion protein according to claim 13 wherein the further peptide is protein A.

15. A kit for assaying for rubella Ig comprising a peptide of any one of claims 1 to 4 or 11 and a signal producing system adapted to interact with the peptide and rubella Ig to provide a detectable signal.


Claims for the following Contracting States: GR, ES

1. A method comprising production of a peptide from the sequence 207 to 353 of Fig. 4 which binds rubella immunoglobulin (Ig) specifically, or of a variant of said peptide which retains rubella Ig specific binding characteristics.

2. A method comprising production of a peptide comprising at least part of the sequence 207 to 353 of Fig. 4 which binds rubella Ig specifically, or of a variant of said peptide which retains rubella Ig specific binding characteristics wherein said peptide is substantially free of natural type glycosylation.

3. A method according to claim 1 or claim 2 comprising production of a peptide from the sequence 245 to 285 of Fig. 4, or of a variant of said peptide which retains rubella Ig specific binding characteristics.

4. A method according to any one of the preceding claims comprising production of a peptide which includes at least part of one or more of the sequences 245-251, 260-266 and 274 to 285 of Fig. 4, or of a variant of said peptide which retains rubella Ig specific binding characteristics.

5. Use of a peptide produced by the method of any one of the preceding claims in the manufacture of a vaccine against rubella.

6. A method for the production of a rubella vaccine said method comprising including a peptide produced by the method of any one of claims to 4 in an injectable formulation.

7. A method of assaying for rubella Ig the method involving use of a peptide produced by the method of any one of claims 1 to 4 as a probe.

8. A method according to claim 7 wherein the peptide is used as an antigen in an ELISA assay specific for IgG or IgM.

9. A method of detecting rubella Ig in a sample medium involving the use of a peptide produced by the method of any one of claims 1 to 4 wherein the peptide carries an enzyme label, said enzyme label catalysing a reaction which leads to a detectable result.

10. A method of detecting rubella Ig in a sample medium involving the use of a peptide produced by the method of any one of claims 1 to 4, said peptide carrying an enzymically inactive small fragment of a primary enzyme; the addition of a complementary enzymically inactive protein fraction of the enzyme so as to conjugate to said small fraction and produce an active primary enzyme; and the performance of a reaction catalysed by said enzyme, leading to a detectable result; and wherein said primary enzyme catalyses a reaction which converts a substrate into a primary product which itself, or as a subsequent product produced by a further reaction or series of reactions initiated by said primary product is an essential component of a second enzyme which is thereby completed and catalyses a reaction leading to a detectable result.

11. An assay for rubella IgM comprising separating total IgM from a sample to be assayed, binding a peptide produced by the method of any one of claims 1 to 4 to rubella IgM in the separated total IgM and detecting the bound peptide by antibody specific to that peptide.

12. A method comprising production of a fusion protein comprising a peptide of any one of claims 1 to 4 and a further peptide.

13. A method according to claim 12 wherein the further peptide is protein A.

14. Use of a peptide produced by the method of any one of claims 1 to 4 in the manufacture of a kit for assaying for rubella Ig said kit comprising, in addition to said peptide, a signal producing system adapted to interact with the peptide and rubella Ig to provide a detectable signal.

12

FIG.1

(a)

| Mc | Peptides (mol. wt. in K daltons) | | | | | | | | |
|---|---|---|---|---|---|---|---|---|---|
| | 13 | 9 | 7.7 | 5 | 4 | 3 | 2.7 | 1.8 | 1.6 |
| EP1 | + | + | + | + | + | − | + | − | − |
| EP2 | + | + | + | − | − | + | − | + | − |
| EP3 | + | + | − | − | − | + | − | − | + |

O.D.
(410 nm)

(b)

O.D.
(410 nm)

11.2K          EP1

EP2

EP3

FRACTION NO.

FIG.2

EP 0 299 673 A1

(a)  Estimated molecular
weight (K)

Peptides

G

13.0
9.0
5.0
4.0
2.7
1.6

G

7.7
3.0
1.8

(b)  Estimated molecular
weight (K)

G

11.2
1.6
1.2
1.0

EP2    EP3    EP1

Epitopes

# FIG.3

GluGluAlaPheThrTyrLeuCysThrAlaProGlyCysAlaThrGln(Thr)ProValPro    20

ValArgLeuAlaGlyValArgPheGluSerLysIleValAspGlyGlyCysPheAlaPro    40

TrpAspLeuGluAlaThrGlyAlaCysIleCysGluIleProThrAspValSerCysGlu    60

GlyLeuGlyAlaTrpValPro(Thr)AlaProCysAlaArgIleTrpAsnGlyThrGlnArg    80

AlaCysThrPheTrpAlaValAsnAlaTyr(Ala)SerGlyGlyTyrAlaGlnLeuAlaSer    100

TyrPheAsnProGlyGlySerTyrTyrLysGlnTyrHisProThrAlaCysGluValGlu    120

ProAlaPheGlyHisSerAspAlaAlaCysTrpGlyPheProThrAspThrValMetSer    140

ValPheAlaLeuAlaSerTyrValGlnHisProHisLysThrValArgValLysPheHis    160

ThrGluThrArgThrValTrpGlnLeuSerValAlaGlyValSerCysAsnValThrThr    180

GluHisProPheCysAsnThrProHisGlyGlnLeuGluValGlnValProProAspPro    200

                         *
GlyAspLeuValGluTyrIleMetAsnTyrThrGlyAsnGlnGlnSerArgTrpGlyLeu    220

GlySerProAsnCysHisGlyProAspTrpAlaSerProValCysGlnArgHisSerPro    240

AspCysSerArg<u>LeuValGlyAlaThrProGluArg</u>ProArgLeuArgLeuValAsp<u>Ala</u>    260
           EP2

<u>AspAspProLeuLeuArg</u>ThrAlaProGlyProGlyGlu<u>ValTrpValThrProValIle</u>    280
   EP3                                        EP1

<u>GlySerGlnAlaArg</u>LysCysGlyLeuHisIleArgAlaGlyProTyrGlyHisAlaThr    300

ValGluMetProGluTrpIleHisAlaHisThr.ThrSerAspProTrpHisProProGly    320

ProLeuGlyLeuLysPheLysThrValArgProValAlaLeuProArg(Ala)LeuAlaPro    340

ProArgAsnValArgValThrGlyCysTyrGlnCysGlyThrProAlaLeuValGluGly    360

LeuAlaProGlyGlyGlyAsnCysHisLeuThrValAsnGlyGlu(Asp)ValGlyAlaVal    380

ProProGlyLysPheValThrAlaAlaLeuLeuAsnThrProProProTyrGlnValSer    400

CysGlyGlyGluSerAspArgAlaThrAlaArgValIleAspProAlaAlaGlnSerPhe    420

ThrGlyValValTyrGlyThrHisThrThrAlaValSerGluThrArgGlnThrTrpAla    440

GluTrpAlaAlaAlaHisTrpTrpGlnLeuThrLeuGlyAla(Val)CysAlaLeu(Leu)Leu    460

AlaGlyLeuLeuAlaCysCysAlaLysCysLeuTyrTyrLeuArgGlyAlaIleAlaPro    480

Arg                    *FIG.4*                                  481

FIG.5

FIG.6

FIG.7

FIG.8

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| X | ARCHIVES OF VIROLOGY, vol. 84, 1985, pages 207-215, Springer-Verlag; L. HO-TERRY et al.: "Rubella virus haemagglutinin: association with a single virion glycoprotein" * Whole document * --- | 1-14 | A 61 K 39/20<br>C 07 K 7/06<br>C 07 K 7/08<br>C 07 K 7/10<br>G 01 N 33/569<br>C 12 N 15/00 |
| D,Y | ARCHIVES OF VIROLOGY, vol. 90, 1986, pages 145-152, Springer-Verlag; L. HO-TERRY et al.: "Immunological characterisation of the rubella E1 glycoprotein" * Whole document * --- | 1-14 | |
| D,Y | CHEMICAL ABSTRACTS, vol. 105, no. 23, 8th December 1986, page 136, abstract no. 203933r, Columbus, Ohio, US; T.K. FREY et al.: "Molecular cloning and sequencing of the region of the rubella virus genome coding for glycoprotein E1", & VIROLOGY 1986, 154(1), 228-32 * Abstract * --- | 1-14 | |
| D,A | CHEMICAL ABSTRACTS, vol. 106, no. 3, 19th January 1987, page 172, abstract no. 13830c, Columbus, Ohio, US; H.L. NAKHASI et al.: "Rubella virus cDNA. Sequence and expression of E1 envelope protein", & J. BIOL. CHEM. 1986, 261(35), 16616-21 * Abstract * --- -/- | | |

TECHNICAL FIELDS
SEARCHED (Int. Cl.4)

C 12 N
A 61 K

The present search report has been drawn up for all claims

| Place of search | Date of completion of the search | Examiner |
|---|---|---|
| THE HAGUE | 07-10-1988 | SKELLY J.M. |

European Patent
Office

**EUROPEAN SEARCH REPORT**

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| A | NUCLEIC ACIDS RESEARCH, vol. 15, no. 7, 10th April 1987, pages 3041-3057, IRL Press Ltd, Oxford, GB; D.M. CLARKE et al.: "Nucleotide sequence and in vitro expression of rubella virus 24S subgenomic messenger RNA encoding the structural proteins E1, E2 and C" | | |
| P,X | ARCHIVES OF VIROLOGY, vol. 98, 1988, pages 189-197, Springer-Verlag; G.M. TERRY et al.: "Localization of the rubella E1 eptiopes" * Whole document * | 1-14 | |
| | | | **TECHNICAL FIELDS SEARCHED (Int. Cl.4)** |

The present search report has been drawn up for all claims

| Place of search | Date of completion of the search | Examiner |
|---|---|---|
| THE HAGUE | 07-10-1988 | SKELLY J.M. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0401)